# EUROPEAN PATENT SPECIFICATION

(11) **EP 1 554 305 B1**
(45) Date of publication and mention of the grant of the patent: **28.03.2007**
(21) Application number: 02779505.3
(22) Date of filing: 23.10.2002
(51) Int. Cl.: C07K 1/04, C07K 5/11, C07K 7/06, C07K 19/00, C12N 15/11, A61K 38/07, A61K 38/08, A61K 39/00, A61P 25/28, G01N 33/68

(54) **PRION PROTEIN-BINDING PEPTIDE SEQUENCES**
PRIONEN-BINDENDE PEPTIDSEQUENZEN
SEQUENCES PEPTIDIQUES LIANT LA PROTEINE PRION

(43) Date of publication of application: 20.07.2005
(73) Proprietor: Centre for Research and Technology Hellas/Institute of Agrobiotechnology IN.A, 570 01 Thermi ,Thessaloniki (GR); Sklaviadis, Theodoros, 570 06 Vasilika (GR); Panagiotides, Cindy, 570 06 Vasilika (GR); Paspaltsis, John, 570 13 Thessaloniki (GR); Karageorgis, Basil, 543 52 Thessaloniki (GR)
(72) Inventor: SKLAVIADIS, Theodoros, Vasilika 57006 (GR); PANAGIOTIDES, Cindy, Vasilika 57006 (GR); PASPALTSIS, John, 57013 Thessaloniki (GR); KARAGEORGIS, Basil, 54352 Thessaloniki (GR)
(74) Representative: Müller-Boré & Partner Patentanwälte
(86) International application number: PCT/EP2002/011868
(87) International publication number: WO 2004/037854

(56) References cited:
- WO-A-00/25814
- WO-A-01/05968
- WO-A-02/44329
- WO-A-02/064734
- US-B1- 6 365 359
- US-B1- 6 399 575
- YEHIELY F ET AL: "IDENTIFICATION OF CANDIDATE PROTEINS BINDING TO PRION PROTEIN" NEUROBIOLOGY OF DISEASE, BLACKWELL SCIENTIFIC PUBLICATIONS, OXFORD, GB, vol. 3, no. 4, 1997, pages 339-355, XP002041124 ISSN: 0969-9961
- SACHSAMANOGLOU M ET AL.: "Antigenic profile of human recombinant PrP: generation and characterization of a versatile polyclonal antiserum" JOURNAL OF NEUROIMMUNOLOGY, vol. 146, no. 1-2, January 2004 (2004-01), pages 22-32, XP002356762

## Description

The present invention refers to proteinaceous compounds that bind to the prion protein and prophylactic and therapeutic uses of these compounds to inhibit the conversion of normal prion protein (PrP^{c}) to the protease-resistant disorder associated isoform, PrP^{sc}.

The prion disorders are a family of fatal neurodegenerative disorders that belong to the group of transmissable subacute spongiform encephalopathies (TSE). At present, Creutzfeldt-Jakob disease (CJD), kuru, fatal familial insomnia syndrome and Gertmann-Straussler-Scheinker disease are the forms that affect humans. Among these, CJD is the most prevalent, affecting about one person per million population per year worldwide (Johnson and Gibbs, (1998) N. Engl. J. Med. 339:1994-2004). Prion disorders of animals include bovine spongiform encephalopathy (BSE) in cows, scrapie in sheep and goats, and chronic wasting disorder of mule deer and elk. BSE, which was first identified in 1986 in the United Kingdom, became a major epidemic in Europe in the 1990s. By April 2001 181,500 BSE-infected cattle had been identified in Europe, predominantly in Great Britain (World Heath Organization, 2001, Official Journal of the European Communities, Special Report No. 14/2001 C324/1 from the court of Auditors, 2001). Outside Europe BSE-infected cattle have been reported in Canada, the Falkland Islands, Oman and, most recently, Japan. It is thought that a new prion disorder, variant CJD, has emerged as a cross-species infection from the consumption of BSE-infected cattle by humans. Over one hundread cases of variant CJD in humans have been diagnosed as of June 2001 (World Health Organizaiton, 2001, *vide supra*). Currently there is no effective treatment or prophylaxis for prion disorders.

A feature common to all TSE diseases is the accumulation of an aberrant isoform of the normal prion protein (denoted PrP or PrP^{c}). The cellular prion protein (PrP^{c}) is a sialoglycoprotein encoded by a gene that in humans is located on chromosome 20 (Oesch, B. et al., Cell 40:735-746, (1985); Basler, K. et al., 46:417-428 (1986); Liao, Y. J. et al., Science 233:364-367 (1986); Meyer, R. K. et al., Proc. Natl. Acad. Sci. USA 83:2310-2314 (1986); Sparkes, R. S. et al., Proc. Natl. Acad. Sci. USA 83:7358-7362 (1986); Bendheim, P. E. et al. J. Infect. Dis. 158:1198-1208 (1988); Turk, E. et al. Eur. J. Biochem. 176:21-30 (1988)). The PrP gene is expressed in neural and non-neural tissues, the highest concentration of mRNA being in neurons (Chesebro, B. et al., Nature 315:331-333 (1985); Kretzschmar, H. A. et al., Am. J. Pathol. 122:1-5 (1986); Brown, H. R. et al., Acta Neuropathol. 80:1-6 (1990); Cashman, N. R. et al., Cell 61:185-192 (1990); Bendheim, P. E., Neurology 42:149-156 (1992)).

The translation product of PrP gene consists of 253 amino acids in humans (Kretzschmar, H. A. et al., DNA 5:315-324 (1986); Pucket, C. et al., Am. J. Hum. 49:320-329 (1991)), 254 in hamster and mice or 256 amino acids in sheep and undergoes several post-translational modifications. In hamsters, a signal peptide of 22 amino acids is cleaved at the N-terminus, 23 amino acids are removed from the C-terminus on addition of a glycosyl phosphatidylinositol (GPI) anchor, and asparagine-linked oligosaccharides are attached to residues 181 and 197 in a loop formed by a disulfide bond (Turk, E. et al., Eur. J. Biochem. 176:21-30 (1988); Hope, J. et al., EMBO J. 5:2591-2597 (1986); Stahl, N. et al., Cell 51:229-240 (1987); Stahl, N. et al., Biochemistry 29:5405-5412 (1990); Safar, J. et al., Proc. Natl. Acad. Sci. USA 87:6377 (1990)).

In prion-related encephalopathies, PrP^{c} is converted into an altered form designated PrP^{sc}, that is distinguishable from PrP^{c} in that PrP^{sc} (i) aggregates; (ii) is proteinase K resistant in that only the N-terminal 67 amino acids are removed by proteinase K digestion under conditions in which PrP^{c} is completely degraded; and (iii) has an alteration in protein conformation from α-helical for PrP^{c} to an altered form (Oesch B. et al., Cell 40:735-746 (1985); Bolton, D. C. et al., Science 218:1309-1311 (1982); McKinley, M. P. et al., Cell 35:57-62 (1982); Bolton, D. C. et al., Biochemistry 23:5898-5905 (1984); Prusiner, S. B. et al., Cell 38:127-134 (1984); Bolton, D. C. et al., Arch. Biochem. Biophys. 258:1515-22 (1987)).

Several lines of evidence suggest that PrP^{sc} may be a key component of the transmissible agent responsible for prion-related encephalopathies (Prusiner, S. B. Science 252:1515-22 (1991)) and it has been established that its protease-resistant core is the major structural protein of amyloid-like fibrils that accumulate intracerebrally in some of these conditions (Brendheim, P. E. et al., Nature 310:418-421 (1984); DeArmond, S. J. et al., Cell 41:221-235 (1985); Kitamoto, T. et al., Ann. Neurol. 20:204-208 (1986); Robert, G. W. et al., N. Engl. Med. 315:1231-1233 (1986); Ghetti, B. et al., Neurology 39:1453-1461 (1989); Tagliavini, F. et al., EMBO J. 10:513-519 (1991); Kitamoto, T. et al., Neurology 41:306-310 (1991)).

To sum up, the abnormal configuration of the prion protein (PrP^{sc}) is considered infectious and resistant to proteolysis (Prusiner, S.B. (1991) Science **252**:1515-1522; Prusiner, S.B. (1998) Proc. Natl. Acad. Sci. USA **95**:13363-13383). Normal PrP protein (PrP^{c}) is expressed in most tissues of the body, with the nervous tissues showing the highest PrP^{c} expression levels (Bendheim et al. (1992), Neurology 42, 149-156, Horiuchi et al, (1995) J. Gen. Virol. 76: 2583-2587) Unlike PrP^{sc} the normal form of PrP is protease sensitive and is not infectious. It is thought, however, that PrP^{sc} is derived from the normal cellular isoform (Prusiner, S.B. (1991) Science **252**:1515-1522). The *in vitro* conversion of PrP^{c} to PrP^{sc} catalyzed by cell-free PrP^{sc} (as initially reported in 1994 by Kocisko,D.A., Come, J.H., Priola, S.A., Chesebro, B., Raynond, C.J., Lansbury, P.T. and Caughey, B. (1994) Nature **370**: 471-474) lends support to this theory.

A prime target for potential therapeutic intervention in neurodegenerative disorders, in particular prion disorders is the conversion of PrP^{c} to PrP^{sc}. A variety of inhibitors of PrP^{sc} formation, including anthracyclines, Congo red, polysulphated glucosminoglycans, and heparins have been examined for possible therapeutic usefulness in the treatment or prevention of prion disorders (Ehlers,B. and Diringer, H. (1984) J. Gen. Virol. **65**:1325-1330.; Caughey, B. and Race, R.E. (1992) J. Neurochem. **59**:768-771.; Caspi, S., Sasson, S.B., Taraboulos, A. and Gabizon, R. (1998) J. Biol. Chem. **273**:3483-3489.; Kimberlin, R.H. and Walker, C.A. (1986) Antimicrob. Agents Chemother. **30**:409-413; Caughey, B. and Raymond, G.J. (1993) J. Virol. **67**:643-650). Inhibitory peptides with homology to PrP have also been suggested as potential therapeutic agents for the treatment of prion disorders (Chesebro, B. et al, US patent application 2001/0041790; Kapumiotou, A. et al, US patent application 2001/0007015 ;Cashman et al, Cashman, N. et al, WO 00/78344; Collinge, J. et al, WO 01/07479; Soto-Jara, C. et al, US Patent 5 948 763). Most recently Prusiner's group screened a panel of drugs known to penetrate the blood brain barrier to determine if they might inhibit PrP^{sc} formation (Korth, C., May, B.C.H., Cohen, F.E. and Prusiner, S.B. (2001) Proc. Natl. Acad. Sci USA **98**:9836-9841). They found two types of drugs, acridine and phenothiazine derivatives, that are fairly potent inhibitors of PrP^{sc} formation in neuroblastoma cells.

One approach to prevention of prion disorders would be a vaccine against PrP. For many bacterial and viral infections vaccines have proven to be quite effective, providing immunity against a variety of disorders to both humans and domestic animals. The poor immunogenicity of PrP, however, has hampered the development of a vaccine effective against prion disorders. However, the potential use of antibodies for prophylaxis of prion disorders is under active investigation in many laboratories. Several groups have reported positive effects for the application of anti-PrP antibodies. Recent developments include the demonstration of both prevention or delays in protease-resistant prion formation in cultured cells (Enari,M. and Weissmann,C. (2001) Proc. Natl. Acad. Sci USA **98**:9295-9299), and in some cases a reversal in the formation of PrP^{sc} in prion infected cells (Peretz, D. et al. (2001) Nature **412**:739-743).

Although a variety of approaches have been taken toward the goal of prevention and/or therapy for prion disorders, there are no existing prophylactic or therapeutic treatments proven to be effective for these disorders in either humans or animals.

Several of the compounds mentioned above have been shown to delay the onset of clinical symptoms and to increase survival times for animals experimentally infected with scrapie, but none has yet been proven to be an effective therapy for prion disorders. Moreover, some of these molecules have such a high risk for potential toxic effects that they would not be appropriate choices for therapeutic agents with which to treat either humans or animals. The toxic side effects can be common toxicity which is for example critical for acridine and phenothiazine. Furthermore, for the peptides and proteins known in the prior art allergic responses as a result of the administration of the drugs are often a severe problem.

Based on the work accomplished to date with cell culture models, the use of antibodies to treat prion disorder has potential, but is still in the early stages of development. There are drawbacks to this therapeutic approach, one being the short half-life of antibodies and another the difficulty of getting antibodies across the blood brain barrier to reach target areas in the brain.

Yehiely F. et al., "Identification of Candidate Binding to Prion Protein", Neurobiology of Disease, 1997, pages 339-355, describes the identification of proteins which bind to PrP by using a fusion protein containing PRP and alkaline phosphatase for screening experiments.

US-patent 6,365,359 describes proteins and functionally equivalent pharmacophores and methods of creating and/or detecting inhibitors of prion formation.

Thus, the technical problem underlying the invention is to provide compounds which both can penetrate the blood brain barrier and are able to bind PrP with high affinity and which therefore can be used as potent inhibitor for the PrP^{sc} formation in the brain. Additionally, a further problem to be solved is to provide compounds with the reduced toxic side effects. This includes especially the reduction of the allergic response upon administering to a patient or animal. A further object of the invention is to provide compounds which are more resistant against enzymatic inactivation.

The above technical problem is solved by the embodiments characterized in the claims.

[The invention particularly refers to a proteinaceous compound consisting of the formula B-[G₃-B]ₓ, wherein each B is independently selected from a group consisting of SEQ ID NO: 1 to 24, G₃ represents three glycine residues, and x is an integer from 1 to 20, said compound being capable to bind to PrP.]

The term "proteinaceous compound" denotes a compound comprising at least one first structural part being an polymer or an oligomer of L-amino acids and/or D-amino acids which are bridged by peptide bonds and one or more optional second parts which do not comprise one or more amino acids such as oligomers or polymers of amino acids and which are linked to the first part(s) by covalent or noncovalent bonds. The amino acids which form the first part include the twenty naturally occurring amino acids, modified amino acids or naturally occurring but rare amino acids. The amino acids can be modified, e.g. by glycosylation. The modifications are usually located at the termini of the first part of the proteinaceous compound. The preferred modifications are an amide group at the C-terminus and/or a formyl or a pyroglutamyl residue at the N-terminus. The so modified proteinaceous compounds may be more stable against the proteolytic attack of carboxypeptidases and aminopeptidases, respectively.

The term "capable to bind to PrP" implies that binding of the respective compound is measurable in the following assay. Bovine serum albumin (BSA), the maltose binding protein (MBP) and a fusion protein of MBP and human PrP are immobilized on a microtiter plate. The compound to be tested is denoted as PrP binding if the binding to the fusion protein significantly exceeds the binding to both BSA and MBP. Unspecific binding is suppressed by blocking with 5 mg/ml BSA, 0.02% NaN₃, 0.1 M NaHCO₃ pH 8.6 and by washing with TBS / 0.5% v/v Tween-20 three times (TBS: 50mM Tris-HCl pH 7.5, 150mM NaCl). If the compound is a peptide, then the assay is performed with 5*10¹¹ phages displaying the peptide instead of the peptide for itself. The expression "capable to bind to PrP" means that the dissociation constant for the binding to PrP is (usually) less than 1 µM, preferably less that 300 nM, more preferably less that 100 nM.

The terms "biological active derivatives" and "functionally active derivative or part thereof' comprise any derivative of a peptide selected form a group consisting of SEQ ID NO: 1 to 24 that is also capable of binding to PrP and, thereby stabilizing PrP against the conformational change to PrP^{sc} and disturbing the concomitant amyloid plaque formation.

The first three sequences SEQ ID NO: 19 to 24 are common PrP binding sequence motifs comprising 5 to 6 amino acids which impart the capability of binding to PrP to proteinaceous compounds and thereby preventing the PrP/PrP^{sc} transition and the concomitant amyloid plaque formation.

The sequences SEQ ID NO: 1 and 12 are a preferred selection for sequence SEQ ID NO: 19, which they comprise, the sequences SEQ ID NO: 2 and 4 are a particularly preferred selection for sequence SEQ ID NO: 20, and similarly the sequences SEQ ID NO: 9 and 15 for sequence SEQ ID NO: 21. The sequences SEQ ID NO: 22 to 24 embrace the sequences according to the SEQ ID NO: 10 and 11.

X denotes a hydrogen bonding amino acid selected from a group consisting of C, W, N, Q, S, T, Y, K, R. H, D, E.

Z is an aromatic amino acid selected from a group consisting of F, W and Y.

The sequences SEQ ID NO: 1 to 24 exhibit a particularly poor immunogenicity.

Proteinaceous compounds comprising these sequences bind very strongly to PrP thereby stabilizing PrP against the conformational change to PrP^{sc} and disturbing the concomitant amyloid plaque formation and preventing, stopping or reversing the development of neurodegenerative disorders, in particular the development of TSE diseases.

Additionally, the specified proteinaceous compounds can easily cross the blood brain barrier. This is a prerequisite for the use of the peptides a therapeutic agents because the amyloid formation to be prevented takes place in the central nervous system.

A further embodiment of the present invention refers to proteinaceous compounds wherein at least one of the amino acid residues is a D-amino acid residue. The incorporation of D-amino acids leads to an improved resistance against proteolytic cleavage without affecting the affinity of binding to the PrP protein. The D-amino acid residue can be located at any position of the proteinaceous compound. A particularly preferred embodiment of the present invention are proteinaceous compounds comprising 3 D-amino acid residues, more preferably 5 D-amino acid residues, most preferably 7 D-amino acid residues.

The proteinaceous compound as defined above consisting of the formula B-[G₃-B]ₓ, wherein each B is independently selected from a group consisting of SEQ ID NO: 1 to 24, G₃ represents three glycine residues (which form a G₃-hinge), and x is an integer from 1 to 20, B can be selected independently, has the following structure if e.g. x=20 :

B⁰-[G₃-B¹]-[G₃-B²] -[G₃-B³] -[G₃-B⁴]-[...]-[G₃-B²⁰],

wherein all B⁰, B¹, B², ... to B²⁰ can have the same or a different meaning. It has been found that these proteinaceous compounds can penetrate the blood brain barrier to a sufficient extent. The availability of the drugs can be further enhanced by using solid colloidal particles named nanoparticles (NP) and microparticles (MP) having a diameter of 30 to 500 nm (NP) or greater than 0,5 µm (MP) as penetration enhancing agent. NPs and MPs are prepared from polylactide polygycolide acid (PGLA), polyalkylcyanoacrylate (PACA) or chitosan and withstand gastric and intestinal fluids. Surfactants, iron transferrin or biological toxins that cross the blood-brain-barrier can be used additionally to facilitate the delivery of drug-loaded NP to the brain. For an example see Schroeder et al. Life Sci. 66, 495-502 (2000).

The resistance of the proteinaceous compounds towards proteolytic cleavage is further improved even in the absence of any modifications or any modified amino acid residues or D-amino acid residues.

On the other hand the affinity of the proteinaceous compound to PrP and the inhibitory effect of the formation of PrP^{sc} and the concomitant amyloid plaque formation is preserved or even improved because of the flexibility of the B-[G₃-B]ₓ group.

Preferably all B⁰, B¹, B², ... to B²⁰ have the same meaning. Because of the fact that in this case the sequence of the first structural part of the proteinaceous compound is highly redundant the immunogenicity is of the proteinaceous compound can be further reduced.

The proteinaceous compounds according to the present invention can be as modified as the peptides known in the art can be, e.g. at the termini by an amid and/or formyl or pyroglutamyl group *(vide supra).*

According to the invention, the proteinaceous compound may be a peptidomimetic compound as a biologically active derivative of a peptide selected form a group consisting of SEQ ID NO: 1 to 24 that is also capable of binding to PrP and, thereby stabilizing PrP against the conformational change to PrP^{sc} and disturbing the concomitant amyloid plaque formation. Peptidomimetic compounds are defined as structures that serve as appropriate substitutes for peptides in interactions with other macromolecules. The discovery of peptidomimetic compounds is feasible by screening of pure compound libraries. Alternatively, computer modeling is used to design analogs of the original molecule. This approach will result in a number of peptide substitutes with improved biostability and the same or improved biological function.

The proteinaceous compounds of present invention may be produced by expressing a nucleic acid comprising a nucleotide sequence encoding the primary amino acid sequence of a compound according to the present invention or a functionally active derivative or part thereof. Functionally active derivatives or parts of the proteinaceous compound are derivatives and parts of the proteinaceous compounds according to the present invention that are capable of binding to PrP. A further embodiment of the present invention is a nucleic acid as defined above. It should be understood that the compounds according to the present invention can also be produced by chemical synthesis.

The proteinaceous compound of present invention may further be used for treating a mammal in need for pharmaceutical prophylactic or therapeutic treatment of a neurodegenerative disorder, especially of a TSE disease. This implies the use of a proteinaceous compound according to the present invention for the manufacture of a medicament for prophylactic or therapeutic treatment of a neurodegenerative disorder, especially of a TSE disease. In more general terms the invention refers to a method of suppressing or reversing the transition of PrP to PrP^{sc} by bringing into contact of either PrP or PrP^{sc} a proteinaceous compound according to the present invention.

As a use of the proteinaceous compound of the present invention for preventing or treating a neurodegenerative disorder or disorder associated with amyloid or amyloid-like deposits said proteinaceous compound according to the present invention is administered in an effective amount to a subject in need thereof, where the subject can be human or animal, in particular a mammal. Likewise, a method of detecting such disorders or disorders also includes administering a sufficient amount of the proteinaceous compound as a probe to visualize its binding to fibril deposits by well known imaging techniques. As the proteinaceous compounds according to the present invention readily penetrate the blood brain barrier the diagnostic method can be performed not only *in vitro* (e.g. by probing slices of the central nervous system with the labeled compound and microscopic investigation of the result) and but also *in vivo* (e.g. by orally administration of the labeled proteinaceous compound and imaging of the distribution of the compounds in the central nervous system).

As used herein, the term "prophylactic treatment" of a condition, such as TSE disease or other amyloidosis disorders, in a subject involves administering a peptide, polypeptide or peptidomimetic compound according to the present invention prior to the clinical onset of the disorder. "Therapeutic treatment" involves administration of a peptide, polypeptide or peptidomimetic compound according to the present invention after the clinical onset of the disorder. For example, successful administration of the peptide of the present invention, after development of a disorder or disorder comprises "therapeutic treatment" of the disorder. The invention is useful in the treatment of humans as well as for veterinary uses in animals.

The proteinaceous compounds according to the present invention may be administered by any means that achieves its intended purpose. For example, administration may be by a number of different parenteral routes including, but not limited to, subcutaneous, intravenous, intradermal, intramuscular, intraperitoneal, intracerebral, intranasal, oral, transdermal, or buccal routes. Parenteral administration can be bolus injection or by gradual perfusion over time.

A typical regimen for preventing, suppressing, or treating a condition associated with amyloid or amyloid-like deposits, comprises either (1) administration of an effective amount in one or two doses of a high concentration of proteinaceous compounds according to the present invention in the range of 0.5 to 10 mg of the respective compound per kg body weight, more preferably 0.5 to 5 mg, or (2) administration of an effective amount of the respective compound administered in multiple doses of lower concentrations in the range of 10-1000 µg per kg body weight, more preferably 50-500 µg over a period of time up to and including several months to several years.

It should be understood that the dosage administered will be dependent upon the age, sex, health, and weight of the recipient, kind of concurrent treatment, if any, frequency of treatment, and the nature of the effect desired. The total dose required for each treatment may be administered by multiple doses or in a single dose. By "effective amount", it is meant a concentration of a proteinaceous compound according to the present invention which is capable of slowing down or inhibiting the formation of amyloid or amyloid-like deposits, or of dissolving preformed fibril deposits. Such concentrations can be routinely determined by those of skill in the art. It will also be appreciated by those of skill in the art that the dosage may be dependent on the stability of the administered proteinaceous compound. A less stable proteinaceous compound may require administration in multiple doses.

Preparations for parenteral administration include sterile aqueous or non-aqueous solutions, suspensions, and emulsions, which may contain auxiliary agents or excipients which are known in the art. Pharmaceutical compositions such as tablets and capsules can also be prepared according to routine methods.

Pharmaceutical compositions comprising the peptides of the invention include all compositions wherein the proteinaceous compounds according to the present invention are contained in an amount effective to achieve its intended purpose. In addition, the pharmaceutical compositions may contain suitable pharmaceutically acceptable carriers comprising excipients and auxiliaries which facilitate processing of the active compounds into preparations which can be used pharmaceutically. Suitable pharmaceutically acceptable vehicles are well known in the art and are described for example in Gennaro, Alfonso, Ed., Remington's Pharmaceutical Sciences, 18th Edition 1990, Mack Publishing Co., Easton, Pa., a standard reference text in this field. Pharmaceutically acceptable vehicles can be routinely selected in accordance with the mode of administration and the solubility and stability of the peptides. For example, formulations for intravenous administration may include sterile aqueous solutions which may also contain buffers, diluents and other suitable additives.

Suitable formulations for parenteral administration include aqueous solutions of the active compounds in water-soluble form, for example, water-soluble salts. In addition, suspension of the active compound as appropriate oily injections suspensions may be administered. Suitable lipophilic solvents or vehicles include fatty oils, for example, sesame oil, or synthetic fatty acid esters for example ethyl oleate or triglycerides. Aqueous injection suspensions that may contain substances which increase the viscosity of the suspension include, for example, sodium carboxymethyl cellulose, sorbitol, and/or dextran. Optionally, the suspension may also contain stabilizers.

Neurodegenerative disorders or disorders associated with abnormal protein folding into amyloid or amyloid-like deposits to be treated or prevented by administering the pharmaceutical composition of the invention include Alzheimer's disorder, FAF, Down's syndrome, other amyloidosis disorders like TSE diseases, prion associated human neurodegenerative disorders such as Kuru, Creutzfeldt-Jakob Disorder (CJD), Gerstmann-Straussler-Scheinker Syndrome (GSS), as well as animal prion disorders such as scrapie, spongiform encephalopathy, transmissible mink encephalopathy and chronic wasting disorder of mule deer and elk.

Besides prophylactic and therapeutic treatments, the respective compounds of the present invention may also be administered to detect and diagnose the presence or absence of amyloid or amyloid-like deposits *in vivo.* A designed peptide capable of binding to structural determinants in a corresponding amyloid or amyloid-like deposit, labeled non-radioactively or with a radioisotope, as is well-known in the art, can be administered to a subject for diagnosing the onset or presence of a disorder or disorder associated with abnormal protein folding into amyloid or amyloid-like fibril deposits. The binding of such a labeled peptide after administration to amyloid or amyloid-like deposits can be detected by *in vivo* imaging techniques known in the art.

The Figures show:

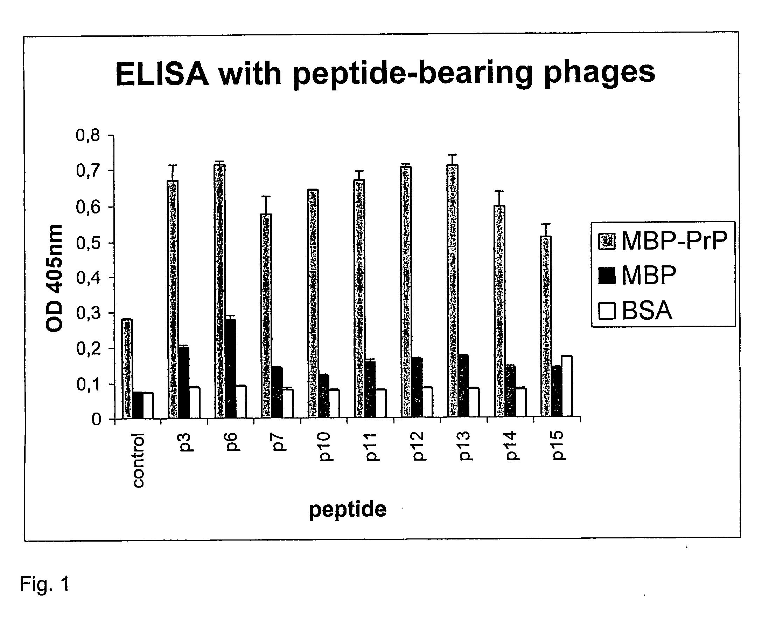

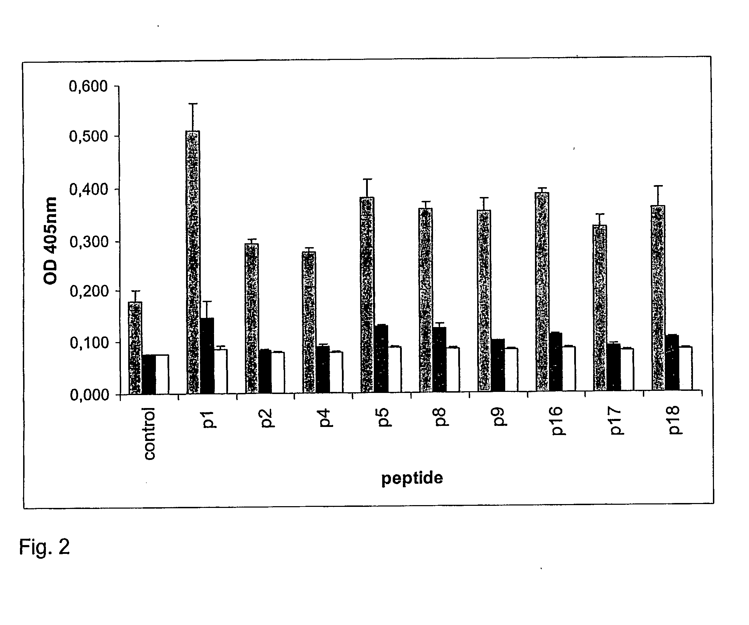

**Fig. 1 and 2** illustrate the results of a binding assay of phage displayed peptides according to the invention and PrP being fused to maltose binding protein (MBP);

| | |
|---|---|
| Ordinate: OD₄₀₅ₙₘ | |
| Abscissa: | |
| The legend reads as follows: | |
| first bar of a bar triplet: | fusion protein of maltose binding protein and PrP |
| second bar of a bar triplet: | maltose binding protein |
| third bar of a bar triplet: | bovine serum albumin |

The peptides are abbreviated as follows:

| | |
|---|---|
| p1 | SEQ ID NO: 1 |
| p2 | SEQ ID NO: 2 |
| p3 | SEQ ID NO: 3 |
| p4 | SEQ ID NO: 4 |
| p5 | SEQ ID NO: 5 |
| p6 | SEQ ID NO: 6 |
| p7 | SEQ ID NO: 7 |
| p8 | SEQ ID NO: 8 |
| p9 | SEQ ID NO: 9 |
| p10 | SEQ ID NO: 10 |
| p11 | SEQ ID NO: 11 |
| p12 | SEQ ID NO: 12 |
| p13 | SEQ ID NO: 13 |
| p14 | SEQ ID NO: 14 |
| p15 | SEQ ID NO: 15 |
| p16 | SEQ ID NO: 16 |
| p17 | SEQ ID NO: 17 |
| p18 | SEQ ID NO: 18 |

The wild type phage which has the normal phage heptapeptide sequence at the N-terminus of its minor coat protein is abbreviated as 'control'.

In Fig. 1 and Fig. 2 peptide-bearing M13-bacteriophage derivatives were assayed for their binding to the indicated proteins, which had been immobilized in individual wells of a microtiter plate. The amount of phage remaining bound after extensive washing was determined by an ELISA assay using a Horseradish peroxidase-coupled monoclonal antibody recognizing the major coat protein of M13 phage (ABTS as substrate to give a green product, the absorbance of which is read at 405 namometers). The experiments were performed as described in Example 2.

Having now generally described the invention, the same will be more readily understood through reference to the following examples which are provided by way of illustration and is not intended to be limiting of the present invention.

### Examples

### Example 1: Isolation of PrP-binding sequences using phage display:

The PhD7 library was purchased from New England Biolabs (California, USA). This library has a diversity of ~2.8x10⁹ random heptapeptides, fused to the N-terminus of the minor coat protein plll of the filamentous bacteriophage M13. For the phage display experiment two polystyrene petri dishes 94x16mm (Greiner, Germany) were coated, the first one with 3.5ml MBP-6xHis 100µg/ml in 0.1 M Na-HCO₃ pH8.6 (MBP: maltose binding protein) and the other one with the same amount and concentration of rhMBP-PrP-6xHis (rh: recombinant human). The coating was performed overnight (>16h) at 4°C with gentle agitation in a humidified container. After coating, the petri dishes were blocked with BSA 5mg/ml (Sigma A-3059) in 0.1M NaHCO₃ pH 8.6 for 1h at 4°C and washed six times with TBST (50mM Tris-HCl pH 7.5, 150mM NaCl, 0.1% v/v Tween-20). 2x10¹¹ pfu (plaque forming units) from the PhD7 library, diluted in 2ml TBST were added to the petri dish coated with MBP-6xHis. After 1h incubation at room temperature with gentle agitation, the supernatant containing the unbound phage was carefully pipetted into the rhMBP-PrP-6xHis coated petri dish and incubated for 1h at room temperature with gentle agitation. Free MBP-6xHis was added to a final concentration of 100µg/ml to block the remaining phages with affinity for MBP-6xHis. The petri dish was then washed ten times with TBST and the bound phage clones were eluted with 2ml 0.2M Glycine pH 2.2, 1mg/ml BSA for 10min and neutralized with 300µl 1 M Tris-HCI pH 9.1. Eluted phages were propagated in the F⁺ E. coli ER2537 strain and titrated according to the manufacturer's recommended instructions. 2x10¹¹ pfu of the amplified phage were used for the second of the five rounds of panning performed. The following rounds of panning were performed in the same way with the following exceptions (1) the incubation time of the phage in the rhMBP-PrP-6xHis coated petri dish was reduced to 30min (2) the concentration of Tween-20 in the TBS was increased to 0.5% v/v.
The second phage display experiment was carried out with the following modifications: (1) 60x15mm polystyrene petri dishes (Greiner, Germany) were used; the volumes of the solutions used were accordingly adjusted (2) the concentration of BSA in the blocking solution was increased to 10mg/ml (3) a two step elution was performed; the phage were eluted specifically first with 1ml rhMBP-PrP-6xHis 100ìg/ml for 1h at room temperature, then non-specifically like described above (4) 3 rounds of panning were performed. DNA of phage clones from the 3^{rd}, 4^{th} and 5^{th} rounds of the first panning experiment and the 2^{nd} and 3^{rd} rounds of the second phage display experiment was isolated according to the manufacturer's recommended instructions and sequenced by MWG-Biotech (Germany).

### Example 2: Characterization of selected phage-presented peptides for PrP binding by ELISA

Flat-bottomed 96-well microtiter plates (Greiner) were prepared for ELISA(Enzyme-linked immunosorbent assay) by pre-coating with either 100µg/ml rhMBP-PrP-6xHis or 100µg/ml MBP-6xHis both in 0.1 M NaHCO₃ pH 8.6 overnight at 4°C in a humified chamber with gentle rocking. Following three washes with TBS/0.5% Tween-20, wells in the plates were subsequently blocked for 2h with the blocking buffer containing 5mg/ml BSA (Sigma A-3059), 0.02% NaN₃ in 0.1M NaHC0₃ pH 8.6. After washing six times with TBS/0.5% Tween-20 the plates were incubated with 10¹² pfu of each of the isolated phage clones obtained from the previous rounds of phage display. After 1 h of incubation at room temperature, unbound phage were subsequently removed by six washes with TBS/0.5% Tween-20. Then the plates were incubated with horseradish peroxidase conjugated anti-M13 monoclonal antibody (Pharmacia # 27-9411-01) 1:5000 in blocking buffer. Following incubation for 1 h at room temperature, plates were washed six times with TBS/0.5% Tween-20 and the bound phage-antibody complex was detected by adding ABTS (Sigma A-1888) solution [0.05M sodium citrate pH 4.0, 0.22mg/ml 2',2'-azino-bis (3'-ethylbenzthiazline-6 sulphonic acid) and 0.05% H₂O₂] and incubated at room temperature for 30min. Absorbance at 405nm was then quantified by ELx800 automated microplate reader (BIO-TEK INSTRUMENTS, INC.). Results for such assays are shown in Figure 1.

## Claims

1. A proteinaceous compound consisting of the formula B-[G₃-B]ₓ, wherein each B is independently selected from a group consisting of SEQ ID NO: 1 to 24, G₃ represents three glycine residues, and x is an integer from 1 to 20, said compound being capable to bind to PrP.

2. The compound according to claim 1, comprising at least one D-amino acid residue.

3. A nucleic acid consisting of a nucleotide sequence encoding the primary amino acid sequence of a compound according to claim 1.

4. A pharmaceutical composition containing a compound as defined in anyone of claims 1 or 2 and optionally a pharmaceutically acceptable carrier.

5. Use of a compound according to one of the claims 1 or 2 for the manufacture of a medicament for the prophylactic or therapeutic treatment of a neurodegenerative disorder.

6. Use according to claim 5, wherein the neurodegenerative disorder is a TSE disease.

7. Use of a compound according to claim 1 or 2 as a probe for the ex vivo diagnosis of a neurodegenerative disorder.

8. Use according to claim 7, wherein the neurodegenerative disorder is a TSE disease.

## Patentansprüche

1. Proteinartige Verbindung, bestehend aus der Formel B-[G₃-B]ₓ, wobei jedes B unabhängig aus der Gruppe, bestehend aus SEQ ID NR.: 1 bis 24 ausgewählt ist, G₃ drei Glycinreste darstellt und x eine ganze Zahl von 1 bis 20 ist, wobei die Verbindung befähigt ist, an PrP zu binden.

2. Verbindung nach Anspruch 1, umfassend mindestens einen D-Aminosäurerest.

3. Nukleinsäure, bestehend aus einer Nukleotidsequenz, welche die primäre Aminosäuresequenz einer Verbindung nach Anspruch 1 kodiert.

4. Pharmazeutische Zusammensetzung, enthaltend eine Verbindung wie in einem der Ansprüche 1 oder 2 definiert und gegebenenfalls einen pharmazeutisch verträglichen Träger.

5. Verwendung einer Verbindung nach einem der Ansprüche 1 oder 2 in der Herstellung eines Medikaments für die prophylaktische oder therapeutische Behandlung einer neurodegenerativen Störung.

6. Verwendung nach Anspruch 5, wobei die neurodegenerative Störung eine TSE-Krankheit ist.

7. Verwendung einer Verbindung nach Anspruch 1 oder 2 als Sonde für die ex vivo Diagnose einer neurodegenerativen Störung.

8. Verwendung nach Anspruch 7, wobei die neurodegenerative Störung eine TSE-Krankheit ist.

## Revendications

1. Composé protéiné présentant la formule B-[G₃-B]ₓ, dans laquelle chaque B est choisi indépendamment dans un groupe formé par les SEQ ID NO : 1 à 24, G₃ représente trois résidus glycine et x est un entier de 1 à 20, ledit composé étant capable de se lier à PrP.

2. Composé selon la revendication 1, comprenant au moins un résidu D-acide aminé.

3. Acide nucléique présentant une séquence de nucléotides codant pour la séquence d'acides aminés primaire d'un composé selon la revendication 1.

4. Composition pharmaceutique contenant un composé tel que défini dans l'une quelconque des revendications 1 ou 2 et éventuellement un support pharmaceutiquement acceptable.

5. Utilisation d'un composé selon l'une quelconque des revendications 1 ou 2 pour la préparation d'un médicament destiné au traitement prophylactique ou thérapeutique d'un trouble de neurodégénérescence.

6. Utilisation selon la revendication 5, dans laquelle le trouble de neurodégénérescence est l'EST.

7. Utilisation d'un composé selon la revendication 1 ou 2 comme sonde pour le diagnostic ex vivo d'un trouble de neurodégénérescence.

8. Utilisation selon la revendication 7, dans laquelle le trouble de neurodégénérescence est l'EST.


### SEQUENCE LISTING

<110> MSP - Molecular Services & Products S.A.
   Centre for Research and Technology Hellas / Institute of Agrobiotechnology
<120> New PrP Binding Heptapeptides
<130>. M4639
<160> 24
<170> PatentIn version 3.1
<210> 1
   <211> 7
   <212> PRT
   <213> Artificial Sequence: obtained by screening a library for PrP binding activity
<400> 1
   Leu Pro Leu Thr Pro Leu Pro
   1               5
<210> 2
   <211> 7
   <212> PRT
   <213> Artificial Sequence: obtained by screening a library for PrP binding activity
<400> 2
   Ser Thr Phe Thr His Pro Arg
   1               5
<210> 3
   <211> 7
   <212> PRT
   <213> Artificial Sequence: obtained by screening a library for PrP binding activity
<400> 3
   Trp Asn Asn Tyr Thr His Pro
   1 5
<210> 4
   <211> 7
   <212> PRT
   <213> Artificial Sequence: obtained by screening a library for PrP binding activity
<400> 4
   His Leu Pro Pro Asn Ser Arg
   1 5
<210> 5
   <211> 7
   <212> PRT
   <213> Artificial Sequence: obtained by screening a library for PrP binding activity
<400> 5
   Phe Gln Pro Pro Pro Asn Pro
   1 5
<210> 6
   <211> 7
   <212> PRT
   <213> Artificial Sequence: obtained by screening a library for PrP binding activity
<400> 6
   Phe Ala Leu Asn Glu Leu Arg
   1 5
<210> 7
   <211> 7
   <212> PRT
   <213> Artificial Sequence: obtained by screening a library for PrP binding activity
<400> 7
   Trp Asn Ser Pro Asn Leu Arg
   1 5
<210> 8
   <211> 7
   <212> PRT
   <213> Artificial Sequence: obtained by screening a library for PrP binding activity
<400> 8
   Ser Val Pro Thr Ile Ile Phe
   1 5
<210> 9
   <211> 7
   <212> PRT
   <213> Artificial Sequence: obtained by screening a library for PrP binding activity
<400> 9
   Ala Thr Thr Arg Thr Leu Pro
   1 5
<210> 10
   <211> 7
   <212> PRT
   <213> Artificial Sequence: obtained by screening a library for PrP binding activity
<400> 10
   Lys Leu Trp Gln Ile Pro Lys
   1 5
<210> 11
   <211> 7
   <212> PRT
   <213> Artificial Sequence: obtained by screening a library for PrP binding activity
<400> 11
   Lys Leu Trp Val Ile Pro Gln
   1 5
<210> 12
   <211> 7
   <212> PRT
   <213> Artificial Sequence: obtained by screening a library for PrP binding activity
<400> 12
   Gly Thr Leu Trp Gln Leu Pro
   1 5
<210> 13
   <211> 7
   <212> PRT
   <213> Artificial Sequence: obtained by screening a library for PrP binding activity
<400> 13
   Lys Cys Cys Tyr Pro Val Pro
   1 5
<210> 14
   <211> 7
   <212> PRT
   <213> Artificial Sequence: obtained by screening a library for PrP binding activity
<400> 14
   Thr Gln Trp Pro Thr Ile Pro
   1 5
<210> 15
   <211> 7
   <212> PRT
   <213> Artificial Sequence: obtained by screening a library for PrP binding activity
<400> 15
   Gln Ala Thr His Arg Ser His
   1 5
<210> 16
   <211> 7
   <212> PRT
   <213> Artificial Sequence: obtained by screening a library for PrP binding activity
<400> 16
   Glu Pro Leu Gln Leu Lys Met
   1 5
<210> 17
   <211> 7
   <212> PRT
   <213> Artificial Sequence: obtained by screening a library for PrP binding activity
<400> 17
   Ala His Pro Gln Leu Ala Thr
   1 5
<210> 18
   <211> 7
   <212> PRT
   <213> Artificial Sequence: obtained by screening a library for PrP binding activity
<400> 18
   His Ala Ile Tyr Pro Arg His
   1 5
<210> 19
   <211> 5
   <212> PRT
   <213> Artificial Sequence: obtained by screening a library for PrP binding activity
<220>
   <221> MISC_FEATURE
   <222> (2)..(3)
   <223> X is a hydrogen bonding amino acid selected from C, W, N, Q, S, T , Y, K, R, H, D, E
<400> 19
   Leu Xaa Xaa Leu Pro
   1 5
<210> 20
   <211> 6
   <212> PRT
   <213> Artificial Sequence: obtained by screening a library for PrP binding activity
<220>
   <221> MISC_FEATURE
   <222> (1)..(2)
   <223> X is a hydrogen bonding amino acid selected from C, W, N, Q, S, T , Y, K, R, H, D, E
<220>
   <221> MISC_FEATURE
   <222> (3).. (3)
   <223> X is an aromatic amino acid selected from F, W, Y
<400> 20
   Xaa Xaa Xaa Thr His Pro
   1 5
<210> 21
   <211> 5
   <212> PRT
   <213> Artificial Sequence: obtained by screening a library for PrP binding activity
<220>
   <221> MISC_FEATURE
   <222> (3).. (3)
   <223> X is a hydrogen bonding amino acid selected from C, W, N, Q, S, T , Y, K, R, H, D, E
<220>
   <221> MISC_FEATURE
   <222> (5)..(5)
   <223> X is a hydrogen bonding amino acid selected from C, W, N, Q, S, T , Y, K, R, H, D, E
<400> 21
   Ala Thr Xaa Arg Xaa
   1 5
<210> 22
   <211> 4
   <212> PRT
   <213> Artificial Sequence: obtained by screening a library for PrP binding activity
<220>
   <221> MISC_FEATURE
   <222> (4) . (4)
   <223> any amino acid (G, A, V, L, I, P, F, Y, W, S, T, N, Q, C, M, D, E , H, K, R)
<400> 22
   Lys Leu Trp Xaa
   1
<210> 23
   <211> 6
   <212> PRT
   <213> Artificial Sequence: obtained by screening a library for PrP binding activity
<220>
   <221> MISC_FEATURE
   <222> (4)..(4)
   <223> any amino acid (G, A, V, L, I, P, F, Y, W, S, T, N, Q, C, M, D, E , H, K, R)
<400> 23
   Lys Leu Trp Xaa Ile Pro
   1 5
<210> 24
   <211> 7
   <212> PRT
   <213> Artificial Sequence: obtained by screening a library for PrP binding activity
<220>
   <221> MISC_FEATURE
   <222> (4)..(4)
   <223> any amino acid (G, A, V, L, I, P, F, Y, W, S, T, N, Q, C, M, D, E , H, K, R)
<220>
   <221> MISC_FEATURE
   <222> (7).. (7)
   <223> polar / hydrophilic amino acid (N, Q, S, T, K, R, H, D, E, C, Y)
<400> 24
   Lys Leu Trp Xaa Ile Pro Xaa
   1 5